# EUROPEAN PATENT SPECIFICATION

(11) **EP 2 807 142 B1**
(45) Date of publication and mention of the grant of the patent: **04.09.2019**
(21) Application number: 13704307.1
(22) Date of filing: 28.01.2013
(51) Int. Cl.: C07C 67/31, C07C 69/73, C07C 253/30, C07C 255/23

(54) **IMPROVED METHYLIDENE MALONATE PROCESS**
VERBESSERTES METHYLIDEN-MALONAT-VERFAHREN
PROCÉDÉ AMÉLIORÉ DE PRODUCTION DE MALONATES DE MÉTHYLIDÈNE

(30) Priority: 28.01.2012 US 201261591884 P
(43) Date of publication of application: 03.12.2014
(73) Proprietor: Optmed Inc., New York, NY 10022 (US)
(72) Inventor: GONDI, Vijaya, Bhasker, Westford, MA 01886 (US); REID, John, Gregory, Groton, MA 01450 (US)
(74) Representative: Patentship Patentanwaltsgesellschaft mbH
(86) International application number: PCT/US2013/023512
(87) International publication number: WO 2013/113035

(56) References cited:
- WO-A1-2008/050313
- WO-A2-2009/053484
- US-A- 4 049 698
- US-B1- 7 718 821
- ATES, C.; ZDENEK, J.; VIEHE, H.: "Trifluoroethylidenation of Compounds with Activated Methylene Groups", TETRAHEDRON LETTERS, vol. 34, no. 36, 31 December 1993 (1993-12-31), pages 5711-5714, XP002694828,

## Description

### FIELD OF THE INVENTION

The present invention relates to an improved process for the production of methylidene malonates.

### BACKGROUND

Methylidene malonates are compounds having the general formula (I): 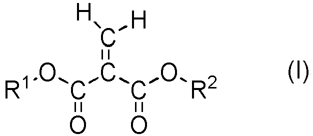 wherein R¹ and R² may be the same or different and represent a C₁ to C₁₈ hydrocarbon group or heterohydrocarbon group having one or more nitrogen, halogen, or oxygen atoms. Such compounds have been known for well over half a century and their use, or potential use, in both organic synthesis and polymer chemistry is well known. Similarly, the use of these compounds as is or as a component of adhesives, including skin bonding adhesive; molding materials; is equally well known. Yet, despite all the promise, these compounds have found limited, If any, commercial success owing to the difficulty of their production; the poor, though improving, yet still erratic, yields; and the general instability of these compounds.

Numerous processes have been developed for the production of methylidene malonates having a formula similar to or falling within the formula of formula (I) above. Two of the earliest methods for the production of methylene dialkyl malonates, the simplest of the methylidene malonates, were the iodide method in which methylene iodide was reacted with dialkyl malonates and the formaldehyde method in which formaldehyde was reacted with dialkyl malonates in the presence of a base, in solution in alcohol solvents. The former was unsatisfactory due to very low yield and expensive starting materials. The latter, though periodically giving better yields than the iodide process, gave relatively poor yields and, more critically, was widely inconsistent from batch to batch, even under the same conditions.

Despite this inconsistency, early efforts continued to focus on the formaldehyde method. One of the most widely practiced formaldehyde methods consisted of reacting diethyl malonate with formaldehyde in glacial acetic acid in the presence of a metal acetate catalyst to produce the diethyl methylidene malonate. The latter was subsequently recovered by distillation following removal of the catalyst by filtration and separating off the solvent. These efforts continued to frustrate and various modifications and iterations of this basic process were developed all in an effort to improve the consistency and yields associated therewith.

Bachman et al. (US 2,313,501) taught the reaction of a C₁-C₅ dialkyl malonate with formaldehyde in the presence of an alkali metal salt of a carboxylic acid, in solution in a substantially anhydrous carboxylic acid solvent, followed by fractional distillation to separate the desired product. Bachman et al. indicate that their process is advantageously carried out in the presence of inhibitors of the polymerization of monomeric vinyl compounds. Suitable inhibitors are said to include the copper salts such as copper chloride and, especially, copper salts of carboxylic acids such as cupric acetate, iron salts such as ferric acetate, and phenols, such as hydroquinone. These are added to the solution mix before the addition of the malonate.

Although Bachman et al. reported yields of up to 72%, the results presented are conversion rates, not yields. Looking at the actual yields of the process, Bachman et al.'s best performance was a yield of 43% with all others being less than 25%. Though Bachman et al. speak of high purity and the ability to recover pure material, they never present any details or data as to what those purities or recoveries were. In any event, Bachman et al. reported that the isolated product, upon standing, polymerized in a day to several weeks time depending upon the purity of the isolated material, which polymer was then heated to facilitate the reversion of the polymer to the monomer.

D'Alelio (US 2,330,033), on the other hand, alleged that such processes were erratic and more often produced yields that averaged 10 to 12 per cent. D'Alelio espoused an improved process with yields on the order of 30% and higher by reacting a malonic acid ester with formaldehyde in a ratio of one mole of the former to at least one mole of the latter under alkaline conditions and, in most cases, in the presence of a polymerization inhibitor such as copper, copper acetate, hydroquinone, resorcinol, or catechol, to form a methylol derivative. The methylol derivative is then acidified to a pH below 7.0 using a suitable organic or inorganic acid in order to retard further reaction. The acidified mass is then dehydrated to form the corresponding methylidene malonate which is subsequently separated by distillation.

Coover et al. (US 3,221,745 and US 3,523,097) took another approach to the formation of the methylidene malonates, electing to begin with a preformed dialkyl alkoxymethylenemalonate. In accordance with their process, the olefinic double bond of the latter compound was subjected to hydrogenation in the presence of a hydrogenation catalyst and the hydrogenated compound was then subject to pyrolysis in the presence of a phosphorous pentoxide inhibitor to strip off the alcohol to produce the methylene malonate. The resultant mass was then subjected to vacuum distillation at low temperature to separate an allegedly high purity methylidene malonate, though with a low yield. According to Coover et al., the use of low temperature distillation is said to prevent the contamination of the monomer with pyrolytic products that commonly result from high temperature distillation. These high purity monomers are said to be especially important for surgical applications.

In discussing the critical need for high purity materials, Coover et al. draw particular attention to the extreme sensitivity of their monomers to the presence of even small amounts of acidic and basic impurities, the former inhibiting polymerization leading to sluggish and ineffective adhesive activity and the latter accelerating polymerization leading to unstable and useless products. They indicate that the amount of such impurities should not exceed 100 ppm, preferably not more than 10 ppm. Unfortunately, other than discussing its limitations with respect to the acidic and basic impurities, and despite its contention of high purity materials, Coover et al. never provide any data pertaining to the purity of their materials. Clearly, though, these materials are not "pure" materials inasmuch as Coover et al., like the others before them and since, require redistillation of the "pure" distillate.

Additionally, although suggesting that their high purity materials "have reasonably good" stability when stored in bulk, they recommend the addition of low levels, 0.0001 to 0.01 weight percent, of a polymerization inhibitor to the monomer materials in order to increase storage stability. Suitable polymerization inhibitors are said to include sulfur dioxide, hydroquinone, nitric oxide, organic acids, boron trifluoride, hydrogen fluoride, stannic chloride, ferric chloride, and organic anhydrides. To assist with cure, particularly cure speed, Coover et al. also suggest the addition of cure accelerators or catalysts to their formulated adhesives, but cautions against adding them too early as they would cause premature polymerization.

Despite the efforts that had been made in advancing the production of methylidene malonates, still no viable method was found. Instability, poor yields, low purity, inconsistency in production, etc. continued to plague this technology. Yet, effort continued and eventually led to multi-step processes in which certain unsaturated molecules served as platforms for the formation of intermediate adducts from which the methylidene malonates were subsequently stripped and recovered. For example, Hawkins et al. (US 4,049,698) found that certain malonic diesters could be reacted with formaldehyde and a linear, conjugated diene in the presence of a primary, secondary or tertiary amine at about reflux temperature to form an intermediate adduct that could then be readily pyrolyzed at temperatures in excess of 600 °C to split off the desired methylidene malonate. Similarly, Ponticello (US 4,056,543) and Ponticello et al. (US 4,160,864) developed processes by which asymmetrical methylene malonates, especially methyl allyl methylene malonate, were prepared from previously formed norbornene adducts, the latter having been prepared by the Diels-Alder reaction of an alkyl acrylate with cyclopentadiene at room temperature or with heating or use of a Lewis catalyst. The so formed monoester norbornene adducts were then reacted with an electrophile material in the presence of an alkyl-substituted lithium amide complex to form the diester adduct and subsequently pyrolyzed at a temperature of 400 °C to 800 °C at a pressure of 1 mm to 760 mm Hg in an inert atmosphere to strip off the desired methylene malonates. These efforts, despite their gains in yield and/or purity, still failed to achieve commercial success.

Citing numerous disadvantages of the foregoing processes, which disadvantages were said to make them difficult, if not impossible, to adapt to industrial scale, Bru-Magniez et al. (US 4,932,584 and US 5,142,098) developed a process whereby anthracene adducts were prepared by reacting mono- or di-malonic acid ester with formaldehyde in the presence of anthracene, most preferably in a non-aqueous solvent medium in the presence of select catalysts. According to Bru-Magniez et al., the anthracene adducts were said to be readily produced in high yields with the desired methylidene malonates obtained by stripping them from the anthracene adduct by any of the known methods including heat treatment, thermolysis, pyrolysis or hydrolysis; preferably heat treatment in the presence of maleic anhydride. The resultant crude products were then subjected to multiple distillations, preferably lower temperature distillations under vacuum, to recover the purified methylidene malonate. Despite the claim to high yields, their crude yields were generally in the range of 21-71%, more importantly, nothing is said with respect to the purity of the material obtained.

Though the Bru-Magniez technology showed promise, instability and inconsistency continued to plague their effort to commercialize these materials. Indeed, owing to the high instability of the overall production process and final products, they reported a high failure rate and of those batches that actually survived through crude distillation, the resultant products had to be stored in a freezer even after stabilizing with upwards of 50,000 ppm SO₂ due to their high instability and spontaneous polymerization.

Malofsky et al. (WO 2010/129068) solved some of the problems associated with process instability of the Bru-Magniez Retro-Diels-Alder adduct process by using select polymerization inhibitor systems in association with the stripping step and, preferably, using the same or different select polymerization inhibitor systems in association with the subsequent isolation and purification steps. Although the stabilizer systems and stabilization techniques of Malofsky et. al. markedly improved the adduct processes, providing greater stability and consistency to the process as well as the final products thereof; these processes still require harsh conditions, including temperatures up to 180 °C; prolonged isolation and purification processes, requiring at least two distillations (three if one employs distillation to remove the crude liquid product from the solids of the reaction mix) to achieve suitable purity; and high costs: the anthracene adduct route is comparatively expensive and while high purities are attained, the repeated purification steps results in overall loss in yield.

More recently, McArdle et al. (WO 2008/050313, U.S. Pat. App. Pub. 2009/0203934, and U.S. Pat. No. 8,022,251) have taught the use certain specific iminium salts, protonated imines, having a tertiary carbon atom attached to a nitrogen atom in the production of electron deficient olefins, most especially cyanoacrylates, however, methylidene malonates are also mentioned. The preferred process involved employing the select iminium salts in producing the 2-cyanoacrylates from nitriles such as ethyl cyanoacetate or malonitrile. When a formaldehyde derivative is used, the McArdle iminium salt can have the structure I: 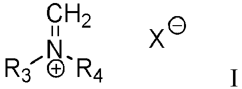 wherein R₃ is H, alkenyl, or alkynyl; R₄ is a hydrocarbon moiety comprising a tertiary carbon which is attached to the N atom, where the tertiary carbon atom is attached to or a part of one or more substituents selected from linear, branched, or cyclic alkyl or alkenyl, or one or more together form a cyclic structure; and X is an anion such as a non-nucleophilic and/or an acidic anion. These imines may be formed by reacting formaldehyde or a source thereof with a primary amine having a tertiary carbon atom attached to the nitrogen to form an imine which is subsequently reacted with an acid under specified conditions to yield an iminium salt. McArdle then employs these iminium salts in the production of electron deficient olefin monomers by reacting the same with certain compounds containing a methylene linkage having at least one electron withdrawing substituent attached thereto where the electron withdrawing substituent is selected from nitrile, carboxylic acids, carboxylic esters, sulphonic acids, ketones and nitro. Employing ethyl cyanoacetate as the certain compound for from 30 minutes to an hour with PRIMENE iminium methane sulfonate resulted in yields and purity of 64%, 90% pure with methanesulfonate anion; 64%, 80% pure with benzenesulfonates anion; 46%, 77% pure with a 1:1 mixture of methanesulfonates and sulfuric acid anions. Similar results, 61%, 80% purity, were attained when the ethyl cyanoacetate was replaced with malononitrile after just 20 minutes reaction time. However, replacement of the ethyl cyanoacetate with dimethyl malonate resulted in yields of only 30% after an hour, with no indication as to purity. Use of a different iminium salt, PRIMENE 81-R iminium-MSA, produced better results, 65% yield and 90% purity with malononitrile, but only a 50% yield of 50% purity when employed with dimethyl malonate.

Variations and refinements of the iminium process are taught in McArdle et al. (U.S. Pat. App. Pub. 2010/0210788) and Bigi et al. (U.S. Pat. No. 7,718,821) where each found generally similar results with their specific iminium salts. Of particular interest in Bigi et. al. is the comparative evaluation of their iminium salt with dimethylmethylideneammonium iodide, also referred to as Eschenmoser's salt, in a reaction with ethyl cyanoacetate. The latter salt is said to have resulted in a low yield, though none is specified, whereas the former gave yields of in excess of 60%: thus, showing the importance of the select iminium salts.

The McArdle et. al. and Bigi et. al. iminium processes are not without their shortcomings. Both require high temperature reactions, temperatures which can promote the *in-situ* polymerization of the monomer product. Additionally, these processes require specific amines to form the iminium salts: amines that are oftentimes expensive and whose reaction byproducts can be difficult to remove. Further, these processes must be conducted at a very low pH in order to prevent the retro-conversion of the iminium salt back to the imine by loss of a proton. From the perspective of the formation of cyanoacrylate monomers, these factors are of low concern, if any, as traditional processes for the production of cyanoacrylates involves the formation of the polymer which is then cracked, typically at high temperature, to form the monomer and have other issues that they too must content with. However, from the perspective of the formation of methylidene malonates, these factors are of considerable concern, particularly inasmuch as the yields and purity of the methylidene malonates so produced, as shown by McArdle et. al., are still low.

In summary, processes for the direct preparation of methylidene malonates from formaldehyde give low yields and inconsistent results. Adduct processes improve yields and purity, but still suffer from instability, inconsistency and high costs. Processes in which select stabilizer systems are employed improves on the instability and inconsistency issues but still suffer from high costs, not just in materials costs and processing time but in terms of the overall yield and purity perspective, especially in view of the need for multiple purification steps which improve purity but lower yield. McArdle and then Bigi provide an alternative direction which may address some of the issues but still suffer from high materials and processing costs and questionable, if not low, yields and purities.

Thus, despite decades and decades of effort and the plethora of processes and process variants and improvements developed over this extended period, there still remains a need for a commercially viable process for the production and isolation of methylidene malonates: a process which balances simplicity of process with common or at least less costly materials with high yields and purity and with consistency and repeatability. There is still a need for a low cost, efficient and consistent process for the production of methylidene malonates in order for these materials to find commercial utility. Specifically, if methylidene malonates are ever to realize their commercial potential and promise, particularly in applications other than niche, high value added applications whose pricing can better offset the losses, costs and low yields of current processes, improved processes must be developed, especially processes that provide for more consistent and predictable yields with high purity and at lower costs.

In following, there is a need for processes for the production of methylidene malonates that are not fraught with process failures, widely varying yields, unstable products, and unintended polymerizations and other by-products such as glutarates and/or dimers, trimers, oligomers and/or polymers of the methylidene malonates. While low levels of the dimers, trimer, oligomers and/or polymers may be tolerated, depending upon the end-use application, it is still best to avoid or certainly lessen their presence in the final products.

Furthermore, there is a need for a process which is suitable for the production of many different methylidene malonates, not just simple dialkyl substituted monomers. In this respect, it is to be appreciated that the use of starting materials wherein the diester of malonic acid contains another functional group, which may be a heteroatom containing functional group, particularly the ester functional group, can adversely affect the production of the corresponding methylidene malonates, making it especially difficult to do so. Similarly, difficulty is encountered where one intends to add or modify such functionality *in-situ* in-process. Additionally, these additional functional groups may promote undesired and competitive reactions to the formation of the methylidene malonate. Thus, there is a need for a general process suitable for the preparation of methylidene malonates from diesters of malonic acid containing one or more other functional groups, especially one or more ester groups.

### SUMMARY OF THE INVENTION

The present invention provides for improved processes for the production of methylidene malonates and for the purification and isolation thereof as well as for the methylidene malonates formed thereby. The improvements provide for a process that produces methylidene malonates quickly and efficiently, in high yield and purity, and at relatively low cost.

According to a first aspect of the present teachings there is provided a method of producing methylidene malonates wherein a malonic acid ester, especially diester, of the formula III: 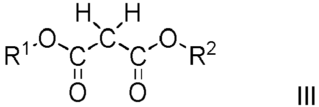 wherein R¹ and R², which may the same or different, are each independently selected from H and a C₁ to C₁₈ hydrocarbon or heterohydrocarbon, the latter having one or more nitrogen, halogen, or oxygen atoms provided that both R¹ and R² are not H, is reacted with an iminium salt of formula II under appropriate conditions, at a temperature in the range of from 0 °C to 60 °C and for an appropriate time period to yield the corresponding methylidene malonate. The iminium salt corresponds to the formula II 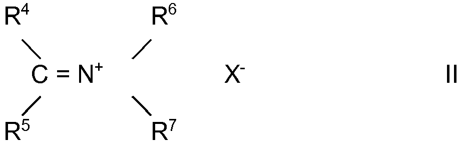 wherein R⁴ and R⁵ are both H, and R⁶ and R⁷ are each a hydrocarbon or substituted hydrocarbon or together form a bridge whereby the nitrogen atom, R⁶ and R⁷ together form a ring structure; provided that neither of R⁶ and R⁷ (inclusive of a ring structure comprising one or both) is a hydrocarbon moiety comprising a tertiary carbon attached to the N atom and X is a halogen, a carboxylate or a sulfonate anion. Preferably, R⁶ and R⁷ are an alkyl, aryl, alkenyl or alkynyl, most preferably alkyl.

Preferred iminium salts are those wherein R⁴ and R⁵ are both H and R⁶ and R⁷ are each independently a hydrocarbon or substituted hydrocarbon moiety, especially an alkyl, aryl, alkenyl or alkynyl moiety, most especially an alkyl moiety, and X is a halogen or a substituted or unsubstituted carboxylate. Especially preferred iminium salts are those wherein R⁴ and R⁵ are hydrogen, and both R⁶ and R⁷ are hydrocarbon moieties, especially alkyl. Most preferred are the dialkyl methylidene ammonium carboxylates, particularly the dialkyl methylidene ammonium acetates and haloacetates.

According to a preferred aspect of the present teachings, the aforementioned iminium salt is formed *in-situ* before being combined with the malonic acid ester or diester. Although one may purify or isolate, in whole or in part (e.g., to concentrate the reaction mix by removing solvent it any), the so formed iminium salt, it is preferable to merely combine the iminium salt reaction product with the malonic acid ester or diester as is. Processes for the production of the iminium salts are well known and fairly simple to prepare. A preferred process involves reacting a tetraaklyldiaminomethane with a strong acylating reagent, especially an acid halide or an acid anhydride, at lowered temperatures. For example, dimethylmethylideneammonium carboxylate is prepared by the reaction of tetramethyldiaminomethane with a carboxylic acid anhydride. Similarly, dimethylmethylideneammonium halide is prepared by the reaction of tetramethyldiaminomethane with an acid halide, especially an acid chloride, most especially a carboxylic acid chloride.

In accordance with the present teachings, the dialkylalkylideneammonium carboxylate or the dialkylalkylideneammonium halide can then be combined with and reacted with a diester of malonic acid, without isolation of the carboxylate or halide salt, to form the desired methylidene malonate monomer. The malonic acid diesters may be symmetric, i.e., both ester groups may the same, or asymmetric with two different ester groups, one or both of which may also include or be substituted with another functional group, especially an ester group.

### DETAILED DESCRIPTION

In accordance with the present teachings there is provided a process, generally an improved process as compared to traditional methods, for the production of methylidene malonates. This process generally comprises the reaction of malonic acid esters and, especially, diesters with certain iminium salts. In accordance with a preferred aspect of the present teachings, there is provided a process, generally an improved process as compared to traditional methods, for the production of methylidene malonates wherein the iminium salt is formed *in-situ,* with or without purification and/or isolation, before combining the same with the malonic acid ester or diester.

Malonic acid esters and diesters are of the formula III: 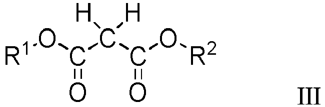 wherein, in the case of the mono-esters, one of R¹ and R² is H and the other a C₁ to C₁₈, preferably C₁ to C₁₂, more preferably C₁ to C₆, hydrocarbon or heterohydrocarbon group, the latter having one or more nitrogen, halogen, or oxygen atoms or, in the case of the diester, both R¹ and R², which may be the same or different, are each independently selected from C₁ to C₁₈, preferably C₁ to C₁₂, more preferably C₁ to C₆, hydrocarbon or heterohydrocarbon groups, the latter having one or more nitrogen, halogen, or oxygen atoms. Preferably R¹ and R² are both hydrocarbon and/or heterohydrocarbon groups and represent a C₁ to C₁₀, more preferably a C₁ to C₆, linear or branched alkyl group; a C₃ to C₆ alicyclic group; a C₂ to C₆ alkenyl group; or a C₂ to C₆ alkynyl group, either or both of which may be substituted with an ether, epoxide, halo, ester, cyano, aldehyde, keto or aryl group. Most preferably, both R¹ and R² are hydrocarbon or heterohydrocarbon groups wherein at least one contains an ester linkage. In this regard, especially desirable diesters of malonic acid are those wherein at least one of the R¹ and R² groups is of the formula IV:

-(CH₂)ₙ - COOR³ IV

wherein R³ is a C₁ to C₁₇, preferably a C₁ to C₆ hydrocarbon or heterohydrocarbon group, the latter having one or more nitrogen, halogen, or oxygen atoms. Preferably, R³ is a C₁ to C₆, preferably a C₁ to C₃, lower alkyl and n is an integer of from 1 to 5, preferably 1 or 2.

Exemplary diesters of malonic acid include dimethyl malonate, diethylmalonate, di-isopropyl malonate, di-n-propyl malonate, and ethyl methyl malonate as well as those of the formula V: 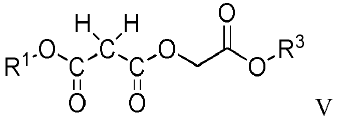 wherein R¹ and R³ are the same or different and represent a C₁ to C₃ lower alkyl, especially ethyl.

The foregoing esters and diesters of malonic acid are reacted with certain iminium salts to form the desired methylidene malonates. The iminium salts correspond to the formula II 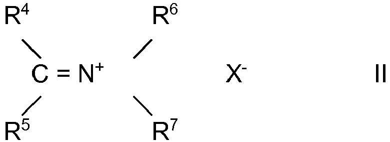 wherein R⁴ and R⁵ are both H, and R⁶ and R⁷ are each a hydrocarbon or substituted hydrocarbon moiety or a hydrocarbon, substituted hydrocarbon or heterohydrocarbon bridge whereby the nitrogen atom, the carbon, or both of formula II are in a ring structure; provided that neither of R⁶ and R⁷ (inclusive of a ring structure comprising one or both) is a hydrocarbon moiety comprising a tertiary carbon attached to the N atom. Generally speaking, if a hydrocarbon or heterohydrocarbon, R⁶ and R⁷ will have from 1 to 10, preferably from 1 to 6 carbon atoms. Preferably, R⁶ and R⁷ are each an alkyl, alkenyl or alkynyl, most preferably alkyl. X is a halogen, a carboxylate or a sulfonate anion.

The iminium salts are those wherein R⁴ and R⁵ are H, and R⁶ and R⁷ are each independently a hydrocarbon or substituted hydrocarbon moiety, especially an alkyl, aryl, alkenyl or alkynyl moiety, most especially a C₁ to C₆ lower alkyl, and X is a halogen or a substituted or unsubstituted carboxylate. Especially preferred iminium salts are those wherein R⁴ and R⁵ are hydrogen and R⁶ and R⁷ are both the same C₁ to C₃ lower alkyl. Most preferred are the dialkyl alkylideneammonium chlorides and carboxylates, particularly the dialkyl alkylideneammonium acetates and haloacetates. For purposes of clarity, "alkyidene" refers to that portion of the iminium compound comprising: 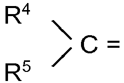 Thus, an iminium compound wherein R⁴ and R⁵ are H and R⁶ and R⁷ are methyl would be referred to as a dimethylmethylidene ammonium compound.

The iminium salts may be formed by a number of alternative processes, all of which are well known in the art. One general route by which they may be formed involves the preparation of the iminium salt from the corresponding imine, which process may further involve the formation of the imine from select amines. Such processes are described in, e.g, Abbaspour Tehrani and De Kimpe, Science of Synthesis, 27, 313 (2004), and references cited therein; Jahn and Schroth, Tett. Lett., 34(37), 5863 (1993); M. B. Smith, Organic Synthesis, McGraw Hill International, Chemistry Series, 1302 (1994) and references cited therein; Hin, B., Majer, P., Tsukamoto, T., J. Org. Chem., 67, 7365 (2002)] and in Mannich reactions [Holy et al, Tetrahedron, 35, 613 (1979); Bryson et al, J. Org Chem., 45, 524 (1980); and McArdle et. al., US 7569,719.

Generally speaking, the iminium salts (also in the past referred to as immonium salts) may be methanimimium salts derived from formaldehyde; ternary iminium salts derived from aldehydes, e.g., acrolein; and quaternary iminium salts derived from ketones. Their preparations may be conducted with or without added catalyst, provided that when a catalyst is added, the catalyst should be one that is not solely a basic nucleophile. Thus, an acidic system would be preferred and a ditropic system may be used, as well.

Typically the imines from which the iminium salts are formed are produced through the reaction of a carbonyl compound, especially an aldehyde, and an amine, such as a primary amine like aniline, N-methylamine, or N-propylamine, which reaction results in the removal of water. Desirably, when a primary amine is used, the primary amine should be one with some degree of steric hindrance, such as tertiary butyl amine. The reaction of primary amine with carbonyl compound is well known and can be a facile, high yielding reaction that may be conducted on a commercial scale e.g., see US 2,582,128 and US 5,744,642.

The so-formed imines from primary amines may be converted into iminium salts by contacting them with an acidic species, such as trifluoroacetic acid, acetic acid, sulphuric acid, methane sulfonic acid, or camphor sulfonic acid.

Another route for preparing the iminium salts is the use of secondary amines wherein a secondary amine, such as dimethylamine, pyrrolidine, morpholine, are first converted to their respective salts and then reacted with the carbonyl compound (with removal of water) to produce iminium salts. Alternatively, the iminium salts can be formed by the reaction of chloromethyl ethers with N-(trimethylsilyl)amines. See e.g. Jahn and Schroth, Tett. Lett., 34(37), 5863 (1993) and Abbaspour Tehrani and De Kimpe, Science of Synthesis, 27, 313 (2004), and references cited therein.

Yet another route for preparing the iminium salts is the direct reaction of certain diamino compounds, such as the 1,1-diaminoalkanes, especially substituted diaminoalkanes, with select activating reagents, especially acid chlorides and acid anhydrides. Such processes are also well known. An especially preferred process of this route employs N,N,N',N'- tetraalkyl-1,1-diaminoaklanes, such as tetramethyldiaminomethane and tetraethyldiaminemethane, as the starting amine.

It is also to be appreciated that many of the suitable iminium salts are available commercially, such as Eschenmoser's chloride and iodide salts which are available from The Aldrich Chemical Co.

Alternatively, and again as noted above, it is also to be appreciated that the iminium salts may also be formed *in-situ,* e.g., as an initial step or series of steps in the production of the methylidene malonates. Specifically, rather than using purchased materials or separately preparing, isolating and purifying the iminium salt, the process of preparing the iminium salt is integrated into the overall methylidene malonate production process. Here the iminium salt is formed (by any of the known methods, especially those noted above) and the malonic acid ester or diester added to the iminium salt, or vice-versa. Depending upon the specific process used to produce the iminium salt, it may be desirable, if not necessary, to isolate or consolidate the so formed iminium salt and/or to remove certain components of the reaction mix, especially catalysts in the case of those processes that employ the same, prior to combining the iminium salt with the malonic acid ester or diester.

Most preferably, it is desired to generate the *in-situ* formed iminium salt using those processes wherein the iminium salt is prepared directly from the reaction of a diamine compound and an activator which contributes the appropriate counter ion, either a halide or a non-nucleophilic conjugate base of an acid. Exemplary anion species include, bromide chloride, iodide, AsF₆, SbF₆, PF₆, BF₄, CH₃SO₃, CF₃SO₃, benzenesulfonate, para-toluenesulfonate, sulfate, bisulfate, perchlorate, SbClₑ, SbCl₃, SnCl₅, carboxylate, and substituted carboxylate. Generally, the amount of diamine to activator to be used in the reaction process is a molar equivalence, though the amine may be used at a slight excess relative to the malonate starting material. Most preferably, though, the activator is employed at a molar excess as compared to the diamine. For example, the molar ratio (activator:diamine) of 1:1 to 10:1, more preferably from 1.2:1 to 5:1 and most preferably from 1.5:1 to 2:1, may be used. These reaction processes occur rapidly and, for the most part, spontaneously: oftentimes requiring cooling to control the exotherm. These reactions are also preferred as the product iminium salt can be used as is and does not require isolation and/or purification.

The preferred iminium salts are the halide salts and the carboxylate salts: though as noted and demonstrated, iminium salts of other anionic species are effective as well. It is also thought that certain benefits may result from the presence of the soft anion (as classified by Pearson's Principles of Hard and Soft Acid Base (HSAB)). In following it is especially preferred that the carboxylate anion is an acetate, a propionate, a pivalate, a stearate, an isobutyrate, or a benzoate; most preferably an acetate.

For purposes of convenience, the present teachings will be discussed in terms of the dialkylmethylideneammonium carboxylate salts, especially the dimethylmethylideneammonium carboxylate salts. However, it is to be appreciated and intended that these teachings are equally applicable to and reflective of the iminium carboxylate salts in general as well as the other iminium salts mentioned above: all of which are suitable for use in the practice of the present process. While not wanting to be bound by theory, it is thought that marked benefit in performance noted with the carboxylate anion, especially in those iminium salts of Formula II above where R⁴ and R⁵ are H and, optionally, though preferably, neither R⁶ nor R⁷ contain a tertiary carbon atom bonded to the N atom, occurs as a result of improved solubility.

The dialkylmethylideneammonium carboxylates may be prepared by a variety of methods. For example, they can be prepared by reacting the desired trialkylamine N-oxide with the acid anhydride of the desired carboxylate anion. Alternatively, the desired dialkylamine can be reacted with formaldehyde or a formaldehyde synthon such as paraformaldehyde in the presence of the carboxylic acid. One preferred method is to prepare the dialkylmethylideneammonium carboxylate by an anion exchange reaction with another more common and, preferably, cheaper, dialkylmethylideneammonium salt such as the commercially available dimethylmethylideneammonium halides, especially the iodide (i.e., Eschenmoser's salt).

More preferably, the dialkylmethylideneammonium carboxylate is prepared by the reaction of tetraalkyldiaminomethane with a carboxylic acid anhydride, e.g., dimethylmethylideneammonium carboxylate is prepared by the reaction of tetramethydiaminomethane with a carboxylic acid anhydride. When this method is employed, the molar ratio of tetraalkyldiaminomethane to carboxylic acid anhydride is preferably from 1:1 to 10:1, more preferably from 1.2:1 to 5:1 and most preferably from 1.5:1 to 2:1. This reaction is preferably conducted in the presence of a solvent such as acetonitrile or toluene. The process is conducted at and, because the reaction is typically exothermic, maintained at a reaction temperature of between 0 °C to 60 °C. Though not critical, it is preferable for reaction control that the carboxylic acid anhydride is added to the tetraalkyldiamino methane as opposed to latter being added to the former. Furthermore, because the reaction is exothermic, it is preferred to perform the addition gradually or in portions and with cooling. Exemplary carboxylic acid anhydrides include acetic anhydride, propionic anhydride, isobutyric anhydride, pivalic anhydride, and benzoic anhydride. Preferably, the carboxylic acid anhydride is acetic anhydride because it is readily available and because any unreacted acetic anhydride and any reaction byproducts such as dimethylacetamide are easily removed. Reaction times vary depending upon the reactants and conditions; however, most often the formation of the iminium salt is completed within a few hours, generally within an hour to an hour and a half. Again, shorter or longer times may be necessary to bring the reaction to completion.

As noted above, the dimethylmethylideneammonium carboxylate may be prepared en-mass or acquired and stored for use. However, for cost convenience and overall simplicity and consolidation of process, it is desirable to employ an *in-situ* formed dimethylmethylideneammonium carboxylate, with or without isolation from its reaction mix. Here, for example, the dimethylmethylideneammonium carboxylate is formed and, without isolation, combined with a diester of malonic acid and allowed to react.

The methylidene malonate is prepared by combining the iminium salt or the in-situ formed iminium salt reaction product with the malonic acid ester or diester. Although either may be added to the other, it is preferable that the diester is added to the iminium salt or iminium salt reaction product. The reaction is performed at a temperature from 0 °C to 60 °C, most typically at room temperature or higher. Higher temperatures can be used and tolerated, but such higher temperatures can result in polymerization or partial polymerization and/or a viscosity increase of the formed methylidene malonate monomer, which results in decreased yields and purity. Similarly, temperatures lower than 0 °C may be used but are not necessary and add to the overall production costs associated with the longer reaction times and the cooling of the reaction system. Furthermore, it is to be appreciated that the specific iminium salt or iminium salt reaction product may also influence the temperature at which the reaction process is carrier out. For example, the presence of excess acid chlorides resulting from the in-situ formation of the halide salts is found to slow the reaction somewhat. Accordingly, elevated temperatures, generally in the range of from 40 °C to 50 °C appear to provide optimal reaction for those salts. Similarly, the reaction appears slower with certain carboxylate salts, again suggesting a desire for elevated temperatures. On the other hand, certain halide salts, such as the Eschenmoser's salts, perform well at room temperature.

The amount of reactants to be employed depends, in part, upon the selected reactants themselves and the impact, if any, of excess on the resultant product or process. Generally speaking, the ratio (on an equivalence basis) of iminium salt to malonic acid ester or diester is from about 1:1 to 10:1, preferably from about 1:1 to 6:1, most preferably, from an economic standpoint, 1:1 to 1:4.

Although not a requirement, it is preferred that the reaction of the diester of malonic acid with the dimethylmethylideneammonium carboxylate is carried out in the presence of a solvent. Indeed, if the iminium salt is formed in-situ, it is preferable that the iminium salt also be prepared in a solvent, most especially the same solvent as is to be employed for the overall reaction. Preferable solvents have a boiling point at atmospheric pressure of between 40° C and 150 °C. Solvents with lower boiling points can cause difficulty and reaction instability because the reaction is exothermic, or in some cases too slow and require heating. Solvents with higher boiling points can be difficult to remove in subsequent purification steps.

The solvents employed may be polar or non-polar solvents. Exemplary polar solvents include DMF, THF, acetonitrile, DMSO, IPA, ethanol. Exemplary non-polar solvents include toluene, benzene, diethylether, hexane, cyclohexane and carbontetrachloride. Polar solvents appear to be optimal for reaction performance in preparing the methylidene malonate; however, pose difficulties in the subsequent work-up to purify and isolate the methylidene malonate. In this respect, it is more difficult to remove the polar by-products from the reaction. On the other hand, non-polar solvents do not provide as optimal a reaction performance as the polar solvents, but make work-up and isolation and purification much simpler and more efficient. It is also to be appreciated that one can conduct the reaction in a polar solvent and then switch the solvent to a non-polar solvent before performing any steps to isolate and/or purify or treat the methylidene malonate monomer. Furthermore, where the 1,1-disubstituted reaction product is heat sensitive, whereby high temperatures polymerize, dimerize and/or degrade the 1,1-disubstituted ethylene monomer produced, it is preferred to use the lower boiling point solvents for those processes where subsequent work-up involves higher temperatures needed for distillation, evaporation, etc., so as to avoid the aforementioned issues.

Generally speaking the amount of solvent to be used is from about 5x to about 30x, preferably about 10x to about 20x, most preferably, on an economic and environmental basis, about 15x to about 20x, the amount of malonic acid ester. Thus, for example, 1 gram of malonic acid ester would be reacted in 20 ml of solvent.

Reaction times for the production of the methylidene malonates will also vary depending upon the reactants, reaction temperature, and the choice of solvent. Reaction times range from under an hour to many hours, indeed 20 or more hours may be necessary to attain complete reaction. Typically, a reaction time of an hour or so up to six hours is suitable and sufficient..

Likewise, though not a requirement, it is preferred that the reaction of the diester of malonic acid with the dimethylmethylideneammonium carboxylate occurs in the presence of an acid or its anhydride, preferably an acid having a pKa less than 6.0, more preferably less than 5.0. The presence of the acid or anhydride is believed to stabilize the methylidene malonate monomer product from polymerization. Suitable acids and anhydrides for preventing the polymerization of methylidene malonate monomers are well known and discussed at length in Malofsky et. al. (US WO 2010/129068). Exemplary acids and anhydrides include acetic acid, acetic anhydride, trifluoroacetic acid, alkyl sulfonic acids such as methanesulfonic acid or trifluoromethanesulfonic acid, arylsulfonic acids such as toluenesulfonic acid, and sulfuric acid. When used, the amount of acid to be added to the reaction mix is preferably from about 100 to about 20,000 ppm, preferably from about 300 to about 10,000 ppm, most preferably from about 2000 to about 5000 ppm based on the amount of the diester of malonic acid. Optimum levels of acid to be added to a given reaction mix can be determined by simple experimentation.

Additional stabilization may be imparted to the reaction mix, especially following or towards the end of the reaction, and/or in association with any subsequent work-up to isolate and/or purify the reaction product, by the addition of one or more free radical polymerization inhibitors. The free radical stabilizer or polymerization inhibitor, as they are more commonly referred, may be added alone or in combination with the acid stabilizer, or any anionic polymerization inhibitor, again as mentioned in Malofsky et. al. Suitable free radical inhibitors include the hydroquinones and various hindered phenols, especially para-hydroquinone monomethyl ether, catechol, pyrogallol, benzoquinones, 2-hydroxy benzoquinones, t-butyl catechol, butylated hydroxy anisole (BHA), butylated hydroxy toluene (BHT), t-butyl hydroquinones, 2,2"-methylene-bis (6-tert-butyl-4-methylphenol), and mixtures thereof. The amount of free radical inhibitor to be added to the system should generally be from about 100 to about 20,000 ppm, preferably from about 300 to about 10,000 ppm, most preferably from about 2000 to about 5000 ppm based on the amount of the diester of malonic acid. As with the acid stabilizer, the optimal amount of free radical polymerization inhibitor to be used can be determined by simple experimentation.

The methylidene malonates formed by the reaction of the ethylene precursors and the iminium salts may be used as-is, but are preferably subjected to various separation, isolation and/or purification steps, all of which are well known in the art. Where the reaction is conducted in a solvent wherein the reaction product is highly soluble therein, it is preferable to replace the solvent with another solvent having no or less solubility properties for the formed methylidene malonate monomer. Again, insofar as isolation and purification of the monomer is concerned, any of the known methods for purification of like organic molecules can be employed; however, purification is preferably achieved by distillation, most preferably under reduced pressure as this allows for lower distillation temperatures. Like the concern with higher reaction temperatures, higher distillation temperatures increase the potential for polymerization or partial polymerization of the methylidene malonate, thereby decreasing the yield.

As noted, it may be desirable to isolate the methylidene malonate material from the reaction mix prior to purification, and especially prior to use. Isolation helps remove unreacted reactants and reaction byproducts. Isolation can be performed by any of the methods known in the art for such purpose. For example, isolation may be conducted as a low temperature distillation under reduced pressure. Alternatively, isolation may be achieved by solvent washing and separation, exemplary solvents include water. Yet another alternative is the treatment of the crude reaction product with a solid adsorbent such as alumina to remove unreacted reactants and reaction byproducts. Preferably, isolation is achieved by a combination of these techniques.

Following the teachings of Malofsky et. al., the isolation and/or purification steps are preferably conducted in the presence of one or more stabilizers/polymerization inhibitors, especially anionic polymerization inhibitors, most especially acid polymerization inhibitors, and/or free radical polymerization inhibitors, most preferably both. Suitable polymerization inhibitors are discussed above and, in more detail, in Malofsky et. al.. Preferably the anionic stabilizer/polymerization inhibitor is an acid stabilizer, most preferably an acid having a pKa less than 2.0. Exemplary acids include trifluoroacetic acid, alkyl sulfonic acids such as methanesulfonic acid or trifluoromethanesulfonic acid, arylsulfonic acids such as toluenesulfonic acid, and sulfuric acid.

Although the stabilizers may be used in any isolation process, they are most preferably used in those isolation processes that involve elevating or elevated temperatures or any other conditions that are known to promote, accelerate or initiate polymerization of methylidene malonate monomer. In any event, stabilizers should, and preferably are, employed in the purification steps, with addition thereof to the distillation pot as well as the collection or receiver vessel. Stabilizers are also to be added to the final collected materials to inhibit polymerization during subsequent storage. Generally speaking the amount of stabilizer (anionic polymerization inhibitor, free radical polymerization inhibitor or both) to be added to the methylididene malonate reaction product, crude reaction product and/or isolated product should be from about 100 to about 20,000 ppm, preferably from about 300 to about 10,000 ppm, most preferably from about 2000 to about 5000 ppm based on the amount of the methylidene malonate. Preferred or optimal stabilizers or combinations of stabilizers as well as the amount thereof to use can be determined by simple experimentation

By implementing the improved processes as set forth herein, one realizes more consistent and improved yields. For example, one may attain crude yields in excess of 50%, preferably in excess of 60%, more preferably in excess of 80%, most preferably in excess of 90%, with purities of, generally, 60% or more, preferably 70% or more, more preferably 80% or more, most preferably 90% or more. Owing to the initial high purity of the crude products, subsequent purification allows for the even higher purity materials with a modest to minimal effect on yield. For example, purified yields in excess of 25%, preferably in excess of 30% with purities of, generally, 90% or more, preferably 95% or more, more preferably 98% and even 99% or more are readily attainable.

Thus, while there may be, and most likely is, some loss in overall yield as a result of the purification process, especially if multiple fractionation processes are employed or the same process is repeated one or more times, the purity of the product significantly improves. This is especially important from a commercial perspective as the purity of the methylidene malonate is has a significant impact upon and correlates with its utility and performance. Specifically, as discussed in Coover et al. (US 3,221,745) and as found by Applicants, even minor amounts of impurities impair their utility, especially the cure or polymerization characteristics of these monomers. Concern with the presence and amount of impurities and byproducts is even more paramount, if not an absolute use limiting factor, in the case of methylidene malonates intended for medical applications, especially skin bonding applications, e.g., skin bonding adhesives, or other applications that may require its use in the human body.

Because the methylidene malonates prepared by the improved process have fewer and different impurities than methylidene malonates prepared by previously known processes, they are more suitable for certain applications. Methylidene malonates prepared in accordance with the present teachings, especially as a result of the reaction of a diester of malonic acid with a dimethylmethylideneammonium carboxylate, are found to have a different impurity profile than those formed from the more traditional methods described in the Background, especially those formed by the adduct processes. Initial studies suggest that these impurities have less of an impact on cure or polymerization and performance, making the resultant methylidene malonates especially suitable for commercial applications, including medical applications.

Perhaps one of the most significant changes and improvements noted in the methylidene malonate reaction products of the present teaching is the absence or at least marked reduction in dimer. The presence of methylidene malonate dimer is and has been a significant impediment to commercial success for these monomers.

In any event, the methylidene malonates formed by the improved process of the present invention may be employed in a number of organic syntheses and polymer chemistry applications. In particular, they are especially useful in the preparation of various adhesive and sealant applications including industrial, commercial and consumer adhesive and sealant applications as well as in medical adhesives, most especially skin bonding applications for human and animal skin bonding. In light of the benefit of the present invention, it is believed that these compositions are now commercially viable as cost effective and stable formulations can now be made.

### EXAMPLES

Having described the invention in general terms, Applicants now turn to the following examples in which specific combinations of reactants, solvents and reaction times were evaluated. These examples are presented as demonstrating the surprising attributes of the improved processes of the present. These examples are merely illustrative of the invention and are not to be deemed limiting thereof.

### Iminium Salts

A plurality of preformed and *in-situ* formed iminium salts were employed. The general structures of these salts were as follows:
Halogen based salts: 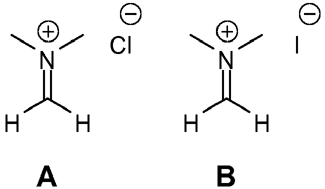
Carboxylate salts: 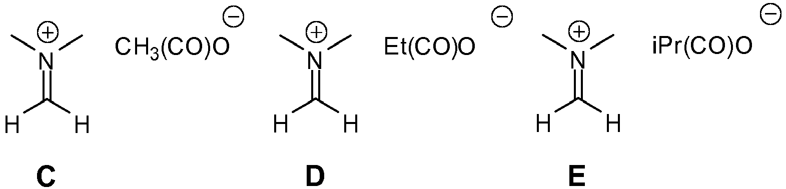 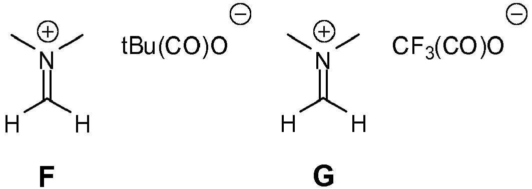 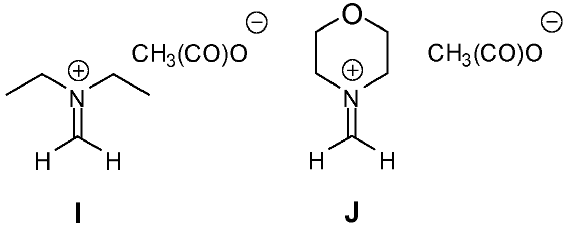
Sulfonate salts:

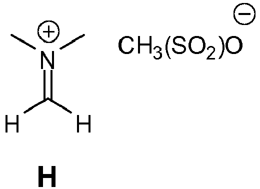

### Monomer

Similarly, three different monomer substrates containing a methylene linkage having attached thereto at least one electron withdrawing group were employed to further demonstrate the breadth of the present teachings as follows: 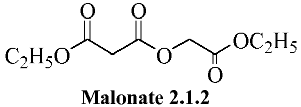 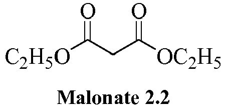 and

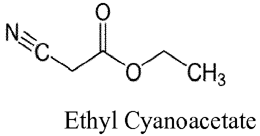

### DMDEE Test

In order to assess cure performance of the 1,1-disubstituted ethylene monomers, a standardized test based on dimorpholinodiethyl ether ("DMDEE") as a cure initiator/activator was developed. This standardized test allowed direct comparison from treated and untreated 1,1-disubstituted ethylene monomers as well as between different types of treatments and variations of the same types of treatments. Specifically, the cure characteristics of 1,1-disubstituted ethylene monomers were assess by inducing the polymerization of the monomers in the presence of DMDEE as follows:

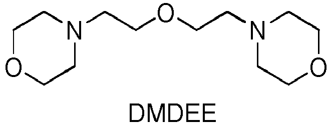

To a tared 4 mL glass vial equipped with a magnetic stir bar, 55 microliters of a 10% by weight solution of DMDEE in isopropanol is added. The vial is reweighed to determine the weight of solution added and monomer is added to the DMDEE solution while stirring to give 1 mL monomer per 42.5 mg DMDEE solution. Stirring is continued for one minute. The stir bar is removed and replaced with a thermocouple. Temperature is plotted versus time. The polymerization induction time is taken as the time in which the rise in temperature between two successive data points (three point running average) first exceeds 0.5 °C. A short induction time is indicative of a monomer that is suitably active for commercial use, i.e., will polymerize in a reasonable period of time. A long induction time is indicative of a monomer that, most likely due to the presence of impurities which inhibit polymerization, that is unsuitable for commercial use owing to the lack of polymerization or a cure speed that is too slow to be of commercial utility.

### Example 1 - Eschenmoser's Iodide Salt (EIS)

6 eq. of EIS (Iminium B) and 0.1 eq. of TFA were added to Malonate 2.1.2 in 20 volumes of 19:1 DMF:IPA solvent. The mixture was stirred for 12-24 hours at room temperature and produced an in-solution yield of -30% of Methylidene Malonate 2.1.2. Analysis of the reaction product showed considerable dimer formation as well.

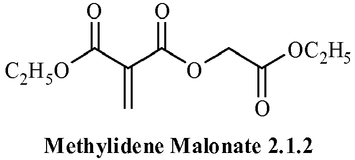

### Example 2 - Reverse Addition

Malonate 2.1.2 dissolved in DMF was slowly added to 6 eq. of EIS (Iminium B) over a period of 2 hours with stirring at room temperature. A measurement was taken after 22 hours and it was found that an in-solution yield of Methylidene Malonate 2.1.2 of 45% had been attained. A further 3 eq. of EIS dissolved in DMF was added after 22 hours and the reaction continued at room temperature for an additional 18 hours. The reaction product then showed an in-solution yield of 47%. Analysis of the reaction product continued to showed considerable dimer formation as well.

### Example 3 - Acid Chloride Addition

Malonate 2.1.2 dissolved in DMF was slowly added to 3 eq. of EIS (Iminium B) over a period of 2 hours with stirring. A measurement was taken after 4 hours and it was found that an in-solution yield of Methylidene Malonate 2.1.2 of 26% had been attained. 0.25 eq. of acetyl chloride was then added to the reaction mix and the reaction continued for an additional 16 hours. The reaction product then showed an in-solution yield of 47%; however, the level of dimer was markedly reduced after the addition of the acetyl chloride, indeed, even lower than was present before the addition of the acetyl chloride. It is theorized that the acid chloride prevents the dimer formation and may actually reverse its formation, possibly via a retro Michael addition.

### Example 4 - Acid Chloride Addition and Higher Temperatures

Having noted that the addition of the acetyl chloride (AcCI) appeared to slow the reaction process at room temperature, another experiment was conducted to consider the impact of higher temperature in combination with the acetyl chloride addition. To correlate the impact of the acetyl chloride and temperature on the reaction and reaction products, both percent conversion of the Malonate 2.1.2 and the percent of Methylidene Malonate 2.1.2 in the reaction products were assessed: dimer typically accounting for sizeable portion of the reaction product. The specific steps and results are presented in Table 1.

**Table 1**

| **Time (hr)** | **Comment** | **Conversion (%)** | **Percentage of Monomer in Products (%)** |
|---|---|---|---|
| 1 | Malonate was added over 1 hr to 3 eq EIS | 46 | 55 |
| 3 | Added 0.25 eq of AcCI after 1 hr | 33 | 73 |
| 6 | Added additional 3 eq of EIS after 3 hrs | 38 | 75 |
| 7 | Started heating to 40 °C | 57 | 73 |
| 21 | After 14 hrs at 40 °C | 62 | 65 |
| 23 | Added additional 0.25 eq AcCl | 57 | 72 |
| 25 | Added additional 3 eq EIS | 61 | 72 |
| 41 | Heated to 50 °C | 85 | 57 |

The results shown in Table 1 clearly demonstrate the marked improvement in yield of the desired Methylidene Malonate 2.1.2 as a result of the addition of the acetyl chloride. Higher temperature appeared to accelerate the conversion; however, elevating the temperature too high with the further addition of EIS led to a loss in the benefit of the acetyl chloride. Presumably, the added EIS led to additional dimer formation and/or degradation of the Methylidene Malonate 2.1.2. Additionally, it is to be recognized that acetyl chloride boils at 52-55 °C and, thus, higher temperatures may lead to a loss in acetyl chloride itself from the reaction pot.

### Example 5 - In-situ Eschenmoser's Chloride Salt (ECS)

A series of experiments were run to assess both the ability to use an *in-situ* formed Eschenmoser's salt, in this case Eschenmoser's chloride salt (ECS - Iminium A), in the production of 1,1-disubstituted ethylenes as well as the impact of varying the mole ratio of the salt forming ingredients on the same. In this specific set of experiments the ECS was formed by the reaction of varying amounts of acetyl chloride and 6 Eq. of tetramethyldiaminomethane (TMDAM) in DMF at 0 °C. Following the formation of the ECS, Malonate 2.1.2 dissolved in DMF was slowly added to the reaction product of the ECS formation and the mixture heated to 60 °C. The specific experiments and the results attained thereby are presented in Table 2.

**Table 2**

| **Run** | **Eq of AcCl** | **Reaction Time (hr)** | **Conversion (%)** | **Percentage of Monomer in Products (%)** |
|---|---|---|---|---|
| 1 | 6.5 | 16 | 98 | 62 |
| 2 | 6.5 | 21 | 99 | 45 |
| 3 | 7.5 | 21 | 99 | 69* |
| 4 | 9 | 25 | 59 | 70 |
| 5 | 12 | 25 | 22 | 55 |

| | | | | |
|---|---|---|---|---|
| * this corresponds to an in-solution yield of Methylidene Malonate 2.1.2 of 68% (% conversion times % Monomer in Products) | | | | |

### Example 6 - Use of Acetonitrile as Solvent

Given the relatively high boiling point of DMF, acetonitrile was evaluated as an alternate polar solvent, as well as to assess whether other polar solvents were suitable. In this experiment 300 ml of acetonitrile was added to a three neck round bottom flash containing 6 eq. TMDAM (56.2 g) and the mixture cooled in an ice bath to 2-3 °C. 7.5 eq. acetyl chloride (48.8 ml) was then added slowly at a rate whereby the temperature of the reaction mix was maintained 20 °C. After the addition was completed, the mixture was removed from the ice bath and allowed to come to room temperature while stirring (∼1 hour). Once at room temperature, 1 eq. of Malonate 2.1.2 monomer (20 g) dissolved in acetonitrile (5 ml) was slowly added. Once the addition was completed, the mixture was stirred for one hour at room temperature and then the mixture heated to 60 °C and stirred for an additional 24 hours. This produced a reaction product with a 97% conversion and an in-solution yield of 80%, as determined by GC.

Once the reaction was complete, the crude reaction mix was cooled to 30 °C and 400 ml of MTBE added to precipitate/crash out the amine salts. The solids were removed by filtration (at continued cold temperature to avoid the salts from going back into solution/melting) and the remaining filtrate was found to have an in-solution yield of 73%.

### Example 7 - Use of Different Acid Chlorides

A further series of experiments were conducted to assess the suitability of various acid chlorides in the iminium process. The same process as employed in Example 6 was employed here as well with the exception of the acid chloride and the mole ratios of the same and the TMDAM. The specific experiments and the results attained thereby are presented in Table 3.

**Table 3**

| **Run** | **TMDAM (eq)** | **Acid Chloride (eq)** | **Time (hr)** | **Conversion (%)** | **In-Solution Yield (%)** |
|---|---|---|---|---|---|
| 1 | 6 | 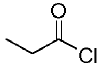 | 18 | 98 | 85 |
| | | 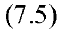 | | | |
| 2 | 3 | 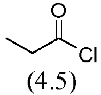 | 20 | >99 | 91 |
| 3 | 2 | 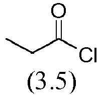 | 20 | 90.5 | 82 |
| 4 | 3 | 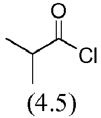 | 20 | 98.6 | 80 |
| 5 | 3 | 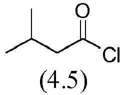 | 20 | 97.5 | 89.9 |
| 6 | 3 | 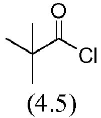 | 20 | 99 | 94.4 |
| 7 | 2 | 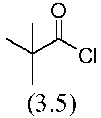 | 20 | 78.5 | - |

Substituting the propionyl chloride (Run 1) for the acetyl chloride (Example 6) while keeping everything else the same presented an immediate jump in yield of ∼5%. The yield jumped even higher when the amount of amine was reduced to 3 eq. and the amount of chloride reduced to 4.5 eq.

### Example 8 - Procedure Using Acetonitrile and Work Up to Remove Ammonium Salts

In this experiment 15 ml of acetonitrile was added to a three neck round bottom flask containing 3 eq. TMDAM (1.4 g) and the mixture cooled in an ice bath to 2-3 °C. 4.5 eq. propionyl chloride (1.8 ml) was then added slowly at a rate whereby the temperature of the reaction mix was maintained below 20 °C. After the addition was completed, the mixture was removed from the ice bath and allowed to come to room temperature while stirring (∼1 hour). Once at room temperature, 1 eq. of Malonate 2.1.2 monomer (1 g) dissolved in acetonitrile (5 ml) was slowly added. Once the addition was completed, the mixture was stirred for one hour at room temperature and then the mixture heated to 60 °C and stirred for an additional 10 hours. This produced a reaction product with an in-solution yield of 91%, as determined by GC.

Once the reaction was complete, the crude reaction mix was concentrated to remove most of the acetonitrile. The remaining reaction product appeared as a viscous oil to which was added toluene and the mixture distilled twice. The mixture was then dissolved in 10 volumes of toluene and an equal amount of MTBE. The resultant slurry was then filtered to remove the solids leaving a solution of the methylidene malonate 2.1.2 monomer at an in-solution yield of 80%.

### Example 9 - Polar and Non-Polar Solvents with Acid Anhydride

A series of experiments were run with polar (acetonitrile) and non-polar (toluene) solvents. The general procedure for both types of solvents is pretty much the same and begins with the addition of 15 volumes of the selected solvent to 2 eq. of the diamine in a round bottom flask kept under nitrogen atmosphere. The reaction mixture is then cooled to 0-5 °C using an ice bath and 3.5 eq. of the acid anhydride is added to the chilled reaction mix at a rate whereby the internal temperature never exceeds 10 °C. After the addition is complete, the mixture is removed from the ice bath and allowed to warm to room temperature, generally over a period of 1-1.5 hours. A solution of the malonate to be converted and 0.1 eq. sulfuric acid or trifluoroacetic avid (TFA) in 5 volumes of the same solvent is then slowly added to the reaction mixture at a rate such that the internal temperature never exceeds 25 °C. Thereafter the processes differ with that process employing the polar solvent continuing to react at room temperature for a few hours, typically less than 6. That process using the non-polar solvent, on the other hand, involved heating the reaction mix to 40 °C and allowing the reaction to continue to completion, generally 15-20 hours.

Table 4 presents the results attained with a number of different acid anhydride derived iminium salts/reaction products in accordance with the general procedure of the preceding paragraph.

**Table 4**

| **Iminium salt** | **Conditions (salt introduction)** | **Solvent** | **In-solution yield (%)** |
|---|---|---|---|
| C | *In-situ* synthesis from acetic anhydride | Acetonitrile | 68 |
| E | *In-situ* synthesis from isobutyric anhydride | Toluene | 27 |
| F | *In-situ* synthesis from trimethylacetic anhydride | Toluene | 89% (conversion) |

### Example 10 - Use of Iminium I

Iminium I was generated from N,N,N',N'-tetraethyldiaminomethane in a manner analogous to the in situ generation of Iminium C (see Example 9). Thus, 2.90 g of N,N,N',N'-tetraethyldiaminomethane was dissolved in acetonitrile (30 mL) and the solution was cooled to 0-5 °C in an ice-water bath. Acetic anhydride (3.28 g) was added, causing the temperature to rise to ∼10 °C. The ice bath was removed and the mixture was stirred for ∼1 hr. The mixture was placed back in an ice-water bath and cooled back to ∼15 °C. A solution of Malonate 2.1.2 (2.00 g) and trifluoroacetic acid ((0.11 g) in acetonitrile (10 mL) was then added and the ice-water bath was again removed. After warming to 20-25 °C, the mixture was stirred for an additional 2 hr, at which point the in-solution yield was measured at 67%.

### Example 11 - Use of Iminium J

Iminium J was generated from N,N',-dimorpholinomethane in a manner analogous to the in situ generation of Iminium I (see Example 10). Thus, 3.42 g of N,N'-dimorpholinomethane was dissolved in acetonitrile (30 mL) and the solution was cooled to 0-5 °C in an ice-water bath. Acetic anhydride (3.28 g) was added, causing the temperature to rise to ∼10 °C. The ice bath was removed and the mixture was stirred for ∼1 hr. The mixture was placed back in an ice-water bath and cooled back to -15 °C. A solution of Malonate 2.1.2 (2.00 g) and trifluoroacetic acid ((0.11 g) in acetonitrile (10 mL) was then added and the ice-water bath was again removed. After warming to 20-25 °C, the mixture was stirred for an additional 2 hr, at which point the in-solution yield was measured at 75%.

## Claims

1. A method of producing methylidene malonates said method comprising reacting a malonic acid ester of the formula 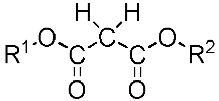 wherein R¹ and R², which may be the same or different, are each independently selected from H and a C₁ to C₁₈ hydrocarbon or heterohydrocarbon, the latter having one or more nitrogen, halogen, or oxygen atoms provided that both R¹ and R² are not H, with an iminium salt of the formula 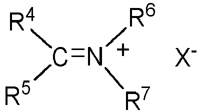 wherein R⁴ and R⁵ are both H and R⁶ and R⁷ are each a hydrocarbon or substituted hydrocarbon or together form a bridge whereby the nitrogen atom, R⁶ and R⁷ together form a ring structure; provided that neither of R⁶ and R⁷ is a hydrocarbon moiety comprising a tertiary carbon attached to the N atom and X is a halogen, a carboxylate or a sulfonate anion, wherein the reaction is conducted at a temperature in the range of from 0 °C to 60 °C.

2. The method of claim 1 wherein R⁶ and R⁷ are each independently alkyl.

3. The method of either of claims 1 or 2 wherein X is a halogen.

4. The method of either of claims 1 or 2 wherein X is a carboxylate.

5. The method of any of claims 1 to 4, wherein R¹ and R² are both hydrocarbon and/or heterohydrocarbon groups and represent a C₁ to C₁₀ linear or branched alkyl group, a C₃ to C₆ alicyclic group, a C₂ to C₆ alkenyl group, or a C₂ to C₆ alkynyl group, either or both of which may be substituted with or contain an ether, epoxide, halo, ester, cyano, aldehyde, keto or aryl group.

6. The method of any of claims 1 to 4, wherein both R¹ and R² are hydrocarbon or heterohydrocarbon groups wherein at least one contains an ester linkage.

7. The method of any of claims 1 to 4, wherein at least one of the R¹ and R² groups is of the formula:
-(CH₂)ₙ - COOR³
wherein R³ is a C₁ to C₁₇ hydrocarbon or heterohydrocarbon group, the latter having one or more nitrogen, halogen, or oxygen atoms, and n is an integer of from 1 to 5.

8. The method of any of claims 1 to 4, wherein the malonic acid ester is a diester and a least one of R¹ and R² is a group is of the formula:
-(CH₂)ₙ - COOR³
wherein R³ is independently a C₁ to C₁₇ hydrocarbon or heterohydrocarbon group, the latter having one or more nitrogen, halogen, or oxygen atoms, and n is an integer of from 1 to 5.

9. The method of either of claims 7 or 8, wherein R³ is independently a C₁ to C₆ lower alkyl and n is 1 or 2.

10. The method of any of claims 1 to 9 wherein the iminium salt is formed in-situ by the reaction of an acid halide or an acid anhydride with a 1,1- diaminoalkane.

11. The method of any of claims 1 to 10 wherein the equivalent weight of iminium salt to malonic acid ester is from 1:1 to 10:1.

12. The method of any of claims 1 to 10 wherein the equivalent weight of iminium salt to malonic acid ester is from 1:1 to 6:1.

13. The method of any of claims 1 to 12 wherein the reaction is performed in the presence of a solvent having a boiling point at atmospheric pressure between 40 °C and 150 °C.

14. The method of claim 13 wherein the solvent is a polar solvent selected from DMF, THF, acetonitrile, DMSO, IPA and ethanol or a non-polar solvent selected from toluene, benzene, diethylether, hexane, cyclohexane, and carbontetrachloride.

15. The method of any of claims 1 to 9 and 11 to 14, wherein the iminium salt is a preformed iminium salt.

## Patentansprüche

1. Verfahren zur Herstellung von Methylidenmalonaten, bei dem man einen Malonsäureester der Formel 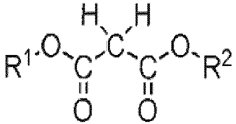 wobei R¹ und R², die gleich oder verschieden sein können, jeweils unabhängig aus H und einem C₁- bis C₁₈-Kohlenwasserstoff oder - Heterokohlenwasserstoff ausgewählt sind, wobei letzterer ein oder mehrere Stickstoff-, Halogen- oder Sauerstoffatome aufweist, mit der Maßgabe, dass nicht R¹ und R² beide für H stehen, mit einem Iminiumsalz der Formel 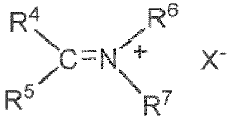 wobei R⁴ und R⁵ beide für H stehen und R⁶ und R⁷ jeweils für einen Kohlenwasserstoff oder substituierten Kohlenwasserstoff stehen oder zusammen eine Brücke bilden, wodurch das Stickstoffatom, R⁶ und R⁷ zusammen eine Ringstruktur bilden, mit der Maßgabe, dass weder R⁶ noch R⁷ für eine Kohlenwasserstoffgruppierung mit einem an das N-Atom gebundenen tertiären Kohlenstoff steht, und X für ein Halogen-, ein Carboxylat- oder ein Sulfonat-Anion steht, umsetzt, wobei die Umsetzung bei einer Temperatur im Bereich von 0 °C bis 60 °C durchgeführt wird.

2. Verfahren nach Anspruch 1, wobei R⁶ und R⁷ jeweils unabhängig für Alkyl stehen.

3. Verfahren nach Anspruch 1 oder 2, wobei X für ein Halogen steht.

4. Verfahren nach Anspruch 1 oder 2, wobei X für ein Carboxylat steht.

5. Verfahren nach einem der Ansprüche 1 bis 4, wobei R¹ und R² beide für Kohlenwasserstoff- und/oder Heterokohlenwasserstoffgruppen stehen und eine lineare oder verzweigte C₁- bis C₁₀-Alkylgruppe, eine C₃- bis C₆-alicyclische Gruppe, eine C₂- bis C₆-Alkenylgruppe oder eine C₂- bis C₆-Alkinylgruppe repräsentieren, wovon eine oder beide durch eine Ether-, Epoxid-, Halogen-, Ester-, Cyano-, Aldehyd-, Keto- oder Arylgruppe substituiert sind oder eine derartige Gruppe enthalten.

6. Verfahren nach einem der Ansprüche 1 bis 4, wobei R¹ und R² beide für Kohlenwasserstoff- und/oder Heterokohlenwasserstoffgruppen stehen, wobei mindestens eine eine Esterbindung enthält.

7. Verfahren nach einem der Ansprüche 1 bis 4, wobei mindestens eine der Gruppen R¹ und R² die Formel:
-(CH₂)ₙ-COOR³
aufweist, wobei R³ für eine C₁- bis C₁₇-Kohlenwasserstoff- oder - Heterokohlenwasserstoffgruppe steht, wobei letztere ein oder mehrere Stickstoff-, Halogen- oder Sauerstoffatome aufweist, und n für eine ganze Zahl von 1 bis 5 steht.

8. Verfahren nach einem der Ansprüche 1 bis 4, wobei es sich bei dem Malonsäureester um einen Diester handelt und mindestens eine von R¹ und R² für eine Gruppe der Formel:
-(CH₂)ₙ-COOR³
steht, wobei R³ unabhängig für eine C₁- bis C₁₇-Kohlenwasserstoff- oder -Heterokohlenwasserstoffgruppe steht, wobei letztere ein oder mehrere Stickstoff-, Halogen- oder Sauerstoffatome aufweist, und n für eine ganze Zahl von 1 bis 5 steht.

9. Verfahren nach Anspruch 7 oder 8, wobei R³ unabhängig für ein C₁-bis C₆-Niederalkyl steht und n für 1 oder 2 steht.

10. Verfahren nach einem der Ansprüche 1 bis 9, wobei das Iminiumsalz durch Umsetzung eines Säurehalogenids oder eines Säureanhydrids mit einem 1,1-Diaminoalkan in situ gebildet wird.

11. Verfahren nach einem der Ansprüche 1 bis 10, wobei das Äquivalentgewicht von Iminiumsalz zu Malonsäureester 1:1 bis 10:1 beträgt.

12. Verfahren nach einem der Ansprüche 1 bis 10, wobei das Äquivalentgewicht von Iminiumsalz zu Malonsäureester 1:1 bis 6:1 beträgt.

13. Verfahren nach einem der Ansprüche 1 bis 12, wobei die Umsetzung in Gegenwart eines Lösungsmittels mit einem Siedepunkt zwischen 40 °C und 150 °C bei Normaldruck durchgeführt wird.

14. Verfahren nach Anspruch 13, wobei es sich bei dem Lösungsmittel um ein polares Lösungsmittel, das aus DMF, THF, Acetonitril, DMSO, IPA und Ethanol ausgewählt ist, oder ein unpolares Lösungsmittel, das aus Toluol, Benzol, Diethylether, Hexan, Cyclohexan und Tetrachlorkohlenstoff ausgewählt ist, handelt.

15. Verfahren nach einem der Ansprüche 1 bis 9 und 11 bis 14, wobei es sich bei dem Iminiumsalz um ein vorgebildetes Iminiumsalz handelt.

## Revendications

1. Procédé de production de malonates de méthylidène, ledit procédé comprenant la réaction d'un ester de l'acide malonique de formule 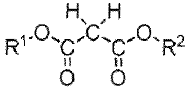 dans lequel R¹ et R², qui peuvent être identiques ou différents, sont chacun indépendamment choisis entre H et un hydrocarbure ou hétérohydrocarbure en C₁ à C₁₈, ce dernier ayant un ou plusieurs atomes d'azote, d'halogène ou d'oxygène, à condition que R¹ et R² ne soient pas tous deux H, avec un sel d'iminium de formule 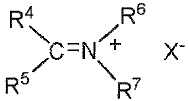 dans lequel R⁴ et R⁵ sont tous deux H et R⁶ et R⁷ sont chacun un hydrocarbure ou un hydrocarbure substitué ou forment ensemble un pont moyennant quoi l'atome d'azote, R⁶ et R⁷ forment ensemble une structure cyclique ; à condition que ni l'un ni l'autre de R⁶ et R⁷ ne soit une fraction hydrocarbonée comprenant un atome de carbone tertiaire lié à l'atome N et X soit un halogène, un carboxylate ou un anion sulfonate, dans lequel la réaction est mise en oeuvre à une température dans la plage de 0 °C à 60 °C.

2. Procédé selon la revendication 1 dans lequel R⁶ et R⁷ sont chacun indépendamment un groupe alkyle.

3. Procédé selon l'une ou l'autre des revendications 1 ou 2 dans lequel X est un halogène.

4. Procédé selon l'une ou l'autre des revendications 1 ou 2 dans lequel X est un carboxylate.

5. Procédé selon l'une quelconque des revendications 1 à 4, dans lequel R¹ et R² sont tous deux des groupes hydrocarbonés et/ou hétérohydrocarbonés et représentent chacun un groupe alkyle en C₁ à C₁₀ linéaire ou ramifié, un groupe alicyclique en C₃ à C₆, un groupe alcényle en C₂ à C₆ ou un groupe alcynyle en C₂ à C₆, l'un ou l'autre de ceux-ci ou les deux pouvant être substitués par un groupe éther, époxyde, halogéno, ester, cyano, aldéhyde, céto ou aryle ou en contenir un.

6. Procédé selon l'une quelconque des revendications 1 à 4, dans lequel R¹ et R² sont tous deux des groupes hydrocarbonés ou hétérohydrocarbonés, au moins l'un de ceux-ci contenant une liaison ester.

7. Procédé selon l'une quelconque des revendications 1 à 4, dans lequel au moins l'un des groupes R¹ et R² répond à la formule :
-(CH₂)ₙ-COOR³
R³ étant un groupe hydrocarboné ou hétérohydrocarboné en C₁ à C₁₇, ce dernier ayant un ou plusieurs atomes d'azote, d'halogène ou d'oxygène, et n étant un nombre entier de 1 à 5.

8. Procédé selon l'une quelconque des revendications 1 à 4, dans lequel l'ester de l'acide malonique est un diester et au moins l'un de R¹ et R² est un groupe de formule :
-(CH₂)ₙ-COOR³
dans lequel R³ est indépendamment un groupe hydrocarboné ou hétérohydrocarboné en C₁ à C₁₇, ce dernier ayant un ou plusieurs atomes d'azote, d'halogène ou d'oxygène, et n est un nombre entier de 1 à 5.

9. Procédé selon l'une ou l'autre des revendications 7 ou 8, dans lequel R³ est indépendamment un groupe alkyle inférieur en C₁ à C₆ et n vaut 1 ou 2.

10. Procédé selon l'une quelconque des revendications 1 à 9 dans lequel le sel d'iminium est formé *in situ* par la réaction d'un halogénure d'acide ou d'un anhydride d'acide avec un 1,1-diaminoalcane.

11. Procédé selon l'une quelconque des revendications 1 à 10 dans lequel le poids équivalent de sel d'iminium par rapport à l'ester de l'acide malonique est de 1:1 à 10:1.

12. Procédé selon l'une quelconque des revendications 1 à 10 dans lequel le poids équivalent de sel d'iminium par rapport à l'ester de l'acide malonique est de 1:1 à 6:1.

13. Procédé selon l'une quelconque des revendications 1 à 12 dans lequel la réaction est effectuée en présence d'un solvant ayant un point d'ébullition à pression atmosphérique compris entre 40 °C et 150 °C.

14. Procédé selon la revendication 13 dans lequel le solvant est un solvant polaire choisi entre le DMF, le THF, l'acétonitrile, le DMSO, l'IPA et l'éthanol ou un solvant non polaire choisi entre le toluène, le benzène, l'oxyde de diéthyle, l'hexane, le cyclohexane et le tétrachlorure de carbone.

15. Procédé selon l'une quelconque des revendications 1 à 9 et 11 à 14, dans lequel le sel d'iminium est un sel d'iminium préalablement formé.
